# EUROPEAN PATENT SPECIFICATION

(11) **EP 0 566 424 B1**
(45) Date of publication and mention of the grant of the patent: **12.02.1997**
(21) Application number: 93302958.9
(22) Date of filing: 16.04.1993
(51) Int. Cl.: B66B 11/02, E04F 15/06

(54) **Elevator platform**
Aufzugskabinenplattform
Plate-forme d'une cabine d'ascenseur

(30) Priority: 16.04.1992 FR 9204703
(43) Date of publication of application: 20.10.1993
(73) Proprietor: OTIS ELEVATOR COMPANY, Farmington, CT 06032 (US)
(72) Inventor: Xiong, Youde, 45500 Gien (FR); Pougny, Jean Pierre, 45500 Saint Gondon (FR); Madrolles, Jacques, 45360 Chatillon Sur Loire (FR)
(74) Representative: Butler, Michael John

(56) References cited:
- WO-A-84/04727
- CH-A- 633 604
- US-A- 4 249 640

## Description

The present invention relates to elevators and more particularly to platforms for supporting a lift or elevator car.

Known platforms for supporting lift or elevator cars generally comprise two portions, a rectangular frame formed by a grid of metallic beams and cross members, and a sheet steel floor secured to an upper surface of the frame by means of screws.

The frame dimensions are determined by the criteria that each of the beams alone must be able to support the entire effective load borne in the elevator. Thus, the beams are all manufactured with the same dimensions. The thickness of the floor is determined on the basis that the load is applied to one quarter of the surface area and that the maximum deformation should be less than 1 mm per meter.

However, a platform of this type has several disadvantages. Since the frame and floor should not bend or deform, it is necessary to add further beams and/or to increase the ability of the frame to carry load without bending. This leads to a heavy structure which increases the energy required to drive the elevator car. Furthermore, the frame must be made by welding which is expensive in itself and which also requires skilled personnel which again adds to the cost. The stresses which are caused by welding are considerable and may produce significant deformation of the platform making mounting of the platform difficult, especially if the platform is large. Finally, at higher welding temperatures, the steel may melt and then buckle and accordingly the platform structure is no longer able to perform its functions.

Other lightweight panels, for use in the aerospace industries, may comprise a continuous low density honeycomb material sandwiched between two outer layers. Such panels are disclosed in WO-A-84/04727 (Boeing) and CH-A-0633604 (Foufounis).

According to one aspect of the present invention, there is provided a platform comprising;
a composite structure including a core comprising a low density material and having a first face and a second face, a protective outer layer secured to the said first face, and a protective outer layer secured to said second face, characterised in that the core is divided into parallel strips that are separated from one another by stakes having the same height as the core.

Thus embodiments of the invention may provide a platform which is simple to manufacture, inexpensive and which has minimal deformation with respect to bending and traction forces as well as impacts. The platform may be for use as an elevator platform.

The platform may thus be provided in the form of a single plate ready for use. This is in contrast to conventional platforms where the frame and floor need to be assembled on site.

The composite platform may be made of materials which are much lighter than the girders used to form the frame of a known elevator platform. The weight of the platform can thus be reduced considerably without affecting the strength thereof. Indeed the mechanical characteristics of the platform may be improved vis-a-vis resistance to bending and impacts, increase in effective life, fire and heat resistance, resistance to piercing, and acoustic insulation.

Finally, the platform may be much easier to produce since the need for welding is dispensed with. Accordingly, fewer skilled personnel are required and the cost of the platform is further reduced.

Embodiments of the invention will now be described by way of example and with reference to the accompanying drawings in which:
Fig. 1 is a perspective view of a platform embodying the present invention;
Fig. 2 is a partial view from above of the platform of Fig. 1;
Fig. 3 is a cross-sectional view along line III-III of Fig. 1;
Fig. 4 is a cross-sectional view along line IV-IV of Fig. 1;
Fig. 5 is an enlarged view in detail of part of Fig. 4;
Fig. 6 is an enlarged view in detail of part of Fig. 3;
Fig. 7 is an enlarged view of one corner of the platform of Fig. 1 with a partially exposed portion;
Fig. 8 is a sectional view along line VIII-VIII of Fig. 7;
Figs. 9 to 12 are examples of inserts used to attach the core of the platform to other elements; and
Fig. 13 is a perspective view of part of a platform of the type shown in Fig. 1, with a honeycomb-shaped core.

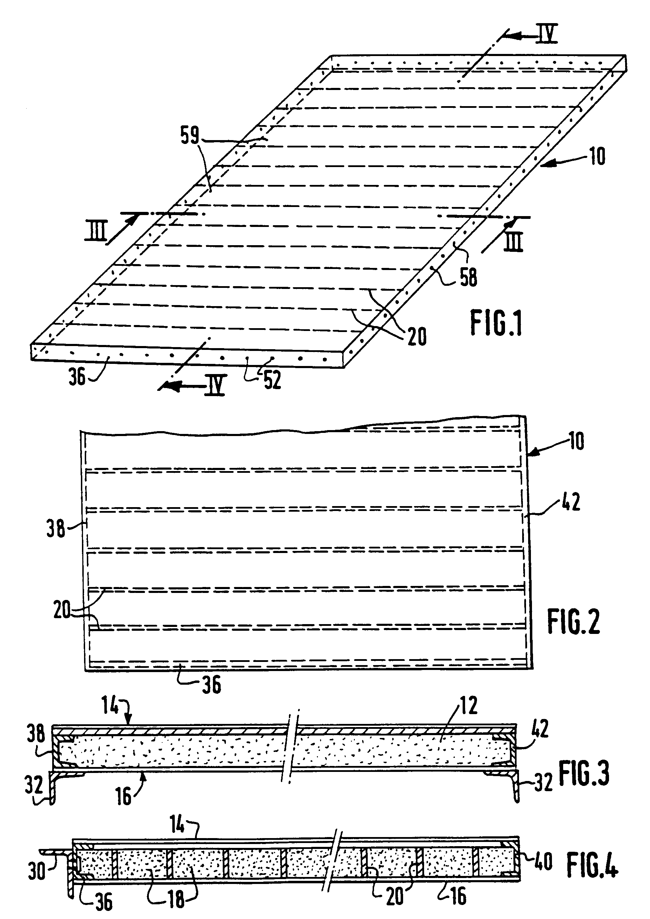

As shown in Figs. 1 and 2, a platform 10 embodying the invention has a generally rectangular shape and has the same dimensions as the internal dimensions of the car of an elevator (not shown).

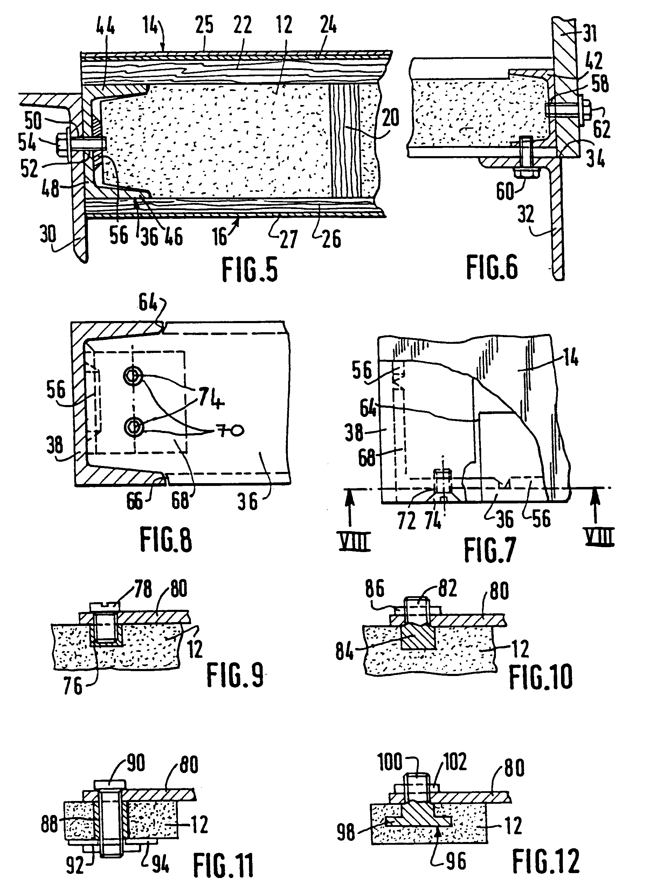

The platform includes a core 12 made of a light weight material sandwiched between two surface coatings or skins 14, 16 (see Fig. 3). The core may be made of any suitable light weight material such as: cardboard; wood; impregnated board; a light metal such as aluminum; or as shown in Figs. 3 and 4, a thermoplastics, such as polyurethane foam.

So as to increase the resistance to crushing or deformation of the platform, the core is divided into parallel strips 18. These strips may for example extend across the width of the platform as shown in Figure 4. The strips 18 are separated from one another by incompressible stakes 20 of wood, cardboard or any other suitable material. The stakes 20 are mounted edgeways to extend over the width of the platform, and have the same height as the core.

As shown in Fig. 5, the upper skin 14, that is the skin orientated towards the top of the platform (i.e. towards a car), has a plywood layer 22 covered externally with a laminated glass fiber and polyester layer 24. A thin metallic plate 25 made, for example, of sheet steel is glued to the exterior of the laminated glass fiber and polyester layer 24 and is provided to improve the resistance of the platform to piercing. The upper layer is intended to provide resistance to piercing and may comprise a thin steel plate such as described above and/or a laminated layer of mineral fibers, such as glass fibers, a fabric mat or other suitable laminated or veneered material.

Since the lower skin 16 is not generally subjected to impacts and piercing forces, it may be less resistant to piercing and/or impact. As shown in Fig. 5, the lower skin has a plywood layer 26 thinner than the plywood layer 22 and covered externally with only one laminated layer of material such as glass fibers 27. A steel plate may however also be glued to the laminated layer 27. The mounting of the protective layers on the core may be effected by any suitable means, such as by gluing or welding.

Secured to the entrance side of the platform are angle irons 30 (see Figs. 4 and 5). Panels 31 (see Fig. 6) are secured to the other three sides, to form the side walls of the car (not shown). Similarly angle irons 32 are fixed to the lower surface 16 of the platform. As shown on Fig. 6, angle irons 32 have a shoulder 34 which serves as a support for the lower edges of the panels 31.

The panels 31 and the angle irons 30 and 32 cannot be secured directly to the core of the platform. This is because screws or the like cannot be attached to alveolar or honeycombed material with sufficient holding strength. To resolve this problem, a frame formed of four metallic U-shaped brackets 36, 38, 40 and 42 is inserted into the four edges of the platform, as shown in Figs. 3 to 6. For example, bracket 36 (Fig. 5) is fixed in such a way that its parallel wings 44, 46 are precisely inserted between the plywood layers 22, 26 and its central section 48 is flush with the edge of the platform. The brackets may also be tubular with a rectangular or square section instead of having the solid section as illustrated in the figures.

Thus, it is possible to attach the angle irons 30, 32 to the brackets. As shown in Fig. 5, for example, the angle iron 30 has a plurality of through-holes 50 placed opposite to tapped holes 52 (see also Fig. 1) provided in the central section 48 of the bracket 36. The angle iron 30 is attached to the bracket by bolts 54 extending through holes 50 and 52. So as to increase the threaded joint thickness, the bracket 36 has a welded flat bar 56 fitted on the internal face of its central section. This flat bar 56 also has tapped holes. The edge of the foam core 12 may be cut to provide space for inserting bracket 36.

Similarly as shown in Figure 6, the bracket 42 has tapped-holes 58 on its central section and tapped holes 59 on its lower wing for screwing the panels 31 and angle irons 32 thereto with bolts 60 and 62 respectively.

Figs. 7 and 8 illustrate a method for mounting two brackets on one corner of the platform, such as the left threshold corner. The threshold bracket 36 has at its extremity two grooves 64 and 66 so that it can be nested at a right angle in the bracket 38. Assembling is effected with the aid of a angle piece 68 whose arms are pierced with tapped holes 70. These holes coincide with the through holes 72 on the central sections of the brackets 36 and 38. These brackets are attached by bolts 74.

The above description describes a method for securing elements to the edges of the platform. When parts need to be secured to the surface of the platform far from the edges, it is necessary to embed in the core 12 threaded or tapped metallic inserts to which said parts are to be secured. Figs. 9 to 12 show several embodiments of inserts.

For example, Fig. 9 shows a nut 76 inserted in the core 12 and a screw 78 screwed into the nut to clamp the part 80 thereto. Fig. 10 shows a screw 82 whose head 84 is embedded in the core 12. Part 80 is fixed to the platform by a nut 86. Fig. 11 shows a metallic sleeve 88 inserted in a hole pierced straight through the thickness of the platform. The part 80 is clamped by a bolt 90 inserted in sleeve 88, a nut 92, and a large diameter washer 94. In Fig. 12, a threaded metallic insert 96 is embedded in the core when the core is molded. The insert 96 comprises a T-shaped head 98 and a threaded rod 100 which projects onto the upper surface of the platform. A nut 102 is screwed onto the threaded rod 100 to clamp part 80 thereto.

A platform embodying the invention may have much better mechanical characteristics than platforms of the prior art. Shear stresses act mainly upon the core 12. To improve shear resistance, the thickness of the core can be increased without significantly increasing the weight of the platform, primarily because of the low density of the core material. Thus, for an equivalent volume, the weight of the platform may be less than that of conventional platform. The cost of the platform is also reduced due to the fact that the materials used for the core are much cheaper that the metallic girders constituting the frame for conventional platforms. Bending tests conducted on a platform of embodying the invention have shown that a deflection of about 0.1 mm/m can be achieved which is clearly less than the standard value of 1mm/m. With laminated composite materials, there is no fatigue limit as there is with steels. The platform can be resistant to impacts resulting from the falling of a load or those resulting from the picking up of a safety catch.

By way of example, a description of two practical embodiments of a platform of embodying the invention is as follows.

### EXAMPLE I - Refer to Figs. 3-6.

### 1. Physical characteristics

| | |
|---|---|
| Dimensions | 2.2 m x 3 m |
| Thickness | 0.1 m |
| Total weight | 500 kg |
| Carrying capacity | 3,200 kg |

A conventional platform having the same dimensions, weighs 1000 kg and costs four times as much.

The materials which may be used are as follows :

| | |
|---|---|
| Steel plate (25) | 3 mm |
| Polyester/glass fiber fabric (24) | 1 mm |
| Finnish birch plywood (22) | 20 mm |
| Polyurethane foam core (12) | 69.5 mm |
| Specific weight of foam | 60 kg/m3 |
| Finnish birch stakes (20) | 69.5 x 20 mm |
| Oregon pine plywood (26) | 9.5 mm |
| Polyester/glass mat laminated plastics (27) | 1 mm |

### 2. Mechanical performances

Loads applied to a surface measuring 6 x 8 cm entail permanent deflections and deformations given in the following table:

| LOAD (in tons): | DEFLECTION (in mm) | PERMANENT DEFORMATION (in mm) |
|---|---|---|
| 2 | 0 | 0 |
| 3 | 0.4 | 0 |
| 4 | 2 | 0 |
| 5 | 3 | start of cracking |

### 3. Comparison of the composite platform and the steel platform

| Characteristics | Composite | Steel |
|---|---|---|
| Resistance to piercing by applying a wheel on a surface of 8 x 6 cm | 3,000 kg | 800 kg |
| Stresses with max load (traction, compression, bending, torsion, buckling) | Plastic deformation 6 mm | Plastic deformation 5 mm |
| Permanent deformation accepted over 1m | 0.1 mm | 1 mm |
| Weight of platform | 500 kg | 1000 kg |
| Thickness of platform | 100 mm | 220 mm |

### EXAMPLE II

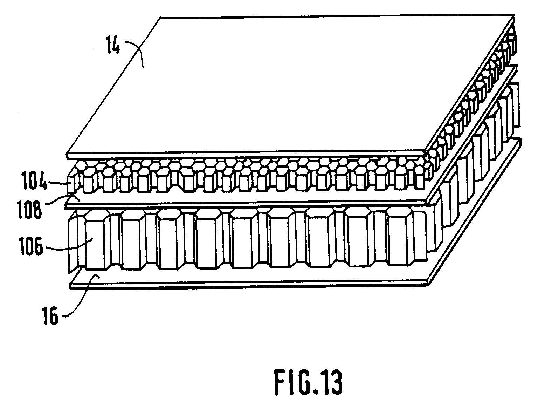

This example is shown by Fig. 13. The core includes two layers 104, 106 having a honeycomb structure and made of aluminum, for example. These layers are separated by steel plate 108. Skins 14, 16 are glued to their external face. This platform exhibits resistance to a particularly high rate of piercing. The upper, more dense honeycomb structure 104 distributes piercing forces over the lower honeycomb layer 106.

## Claims

1. A platform (10) comprising;
a composite structure including a core (12) comprising a low density material and having a first face and a second face, a protective outer layer (14) secured to the said first face, and a protective outer layer (16) secured to said second face, characterised in that the core is divided into parallel strips (18) that are separated from one another by stakes (20) having the same height as the core.

2. A platform as claimed in claim 1, wherein the core (12) is comprised of any one of wood, thermoplastics foam, impregnated cardboard or a low density metal.

3. A platform as claimed in claim 1 or 2, wherein said stakes (20) are of substantially incompressible material.

4. A platform as claimed in claim 1 or 2, wherein the core comprises two layers (104,106), at least one of which has a honeycomb structure (108), said layers being attached to an intermediate plate.

5. A platform as claimed in any preceding claim, wherein at least one of the protective layers (14,16) is formed of a laminated composite material.

6. A platform as claimed in claim 5, wherein at least one of the protective layers is a laminated sheet comprising an inner fiber mat (24) and an outer thin metallic plate (26).

7. A platform as claimed in any preceding claim, comprising a peripheral frame (36,38,40,42) inserted between the protective layers (14,16), said frame having a portion flush with edges of the platform (10).

8. A platform as claimed in claim 7, wherein an attachment (30,31) is securable to said flush portion of said frame.

9. A platform as claimed in claim 7 or 8, wherein an attachment (32,80) is securable to the frame through one of the protective layers of the platform.

10. A platform as claimed in claim 8 or 9, wherein an elevator cab, in use, rests upon said attachment (30).

11. A platform as claimed in any preceding claim, wherein inserts (76,82,88,96) for attaching parts to said platform (10) are embedded in said core (12) and extend through at least one of said protective layers (14,16).

12. A platform as claimed in any preceding claim, wherein the platform (10) is suitable for use as an elevator platform.

## Patentansprüche

1. Plattform (10), aufweisend
eine Verbundstruktur, die einen Kern (12) aus einem Material geringer Dichte mit einer ersten Fläche und einer zweiten Fläche, eine an der ersten Fläche befestigte äußere Schutzshicht (14) und eine an der zweiten Fläche befestigte äußere Schutzchicht (16) aufweist,
dadurch gekennzeichnet,
daß der Kern in parallele Streifen (18) geteilt ist, die voreinander durch Stege (20) getrennt sind, die die gleiche Höhe wie der Kern besitzen.

2. Plattform nach Anspruch 1, bei welcher der Kern (12) aus einem der folgenden Materialien besteht: Holz, thermoplastischer Schaum, imprägnierter Karton oder einem Metall geringer Dichte.

3. Plattform nach Anspruch 1 oder 2, bei der die Stege (20) aus einem im wesentlichen inkompressiblen Material bestehen.

4. Plattform nach Anspruch 1 oder 2, bei welcher der Kern zwei Schichten (104, 106) aufweist, von denen mindestens eine eine wabenartige Struktur (108) besitzt, wobei die Schichten an einer Zwischenplatte befestigt sind.

5. Plattform nach einem der vorhergehenden Ansprüche, bei der mindestens eine der Schutzschichten (14, 16) aus einem laminierten Verbundmaterial gebildet ist.

6. Plattform nach Anspruch 5, bei der mindestens eine der Schutzschichten eine laminierte Tafel ist, die eine innenliegende Fasermatte (24) und ein außenliegende dünne Metallplatte (26) aufweist.

7. Plattform nach einem der vorhergehenden Ansprüche, aufweisend einen zwischen die Schutzschichten (14, 16) eingesetzten Umfangsrahmen (36, 38, 40, 42), der einen mit den Rändern der Plattform (10) bündigen Bereich besitzt.

8. Plattform nach Anspruch 7, bei der ein Ansatzteil (30, 31) an dem bündigen Bereich des Rahmens befestigt werden kann.

9. Plattform nach Anspruch 7 oder 8, bei der ein Ansatzteil (32, 80) durch eine der Schutzschichten der Plattform hindurch an dem Rahmen befestigt werden kann.

10. Plattform nach Anspruch 8 oder 9, bei der eine Aufzugskabine im Betrieb auf dem Ansatzteil (30) aufliegt.

11. Plattform nach einem der vorhergehenden Ansprüche, bei der Einsätze (76, 82, 88, 96) zum Anbringen von Teilen an der Plattform in dem Kern (12) eingebettet sind und sich durch mindestens eine der Schutzschichten (14, 16) erstrecken.

12. Plattform nach einem der vorhergehenden Ansprüche, wobei die Plattform (10) für die Verwendung als Aufzugsplattform geeignet ist.

## Revendications

1. Plate-forme (10) comprenant :
une structure composite incluant une âme (12) réalisée en un matériau de faible densité et ayant une première face et une seconde face, une couche de revêtement extérieure (14) fixée sur ladite première face, et une couche de revêtement extérieure (16) fixée sur ladite seconde face, caractérisée en ce que l'âme est divisée en bandes parallèles (18) qui sont séparées les unes des autres par des tasseaux (20) qui ont la même hauteur que l'âme.

2. Plate-forme selon la revendication 1, dans laquelle l'âme (12) est réalisée en l'une quelconque des matières comprenant le bois, une mousse de matière thermoplastique, le carton imprégné ou un métal de faible densité.

3. Plate-forme selon la revendication 1 ou 2, dans laquelle lesdits tasseaux (20) sont en une matière pratiquement incompressible.

4. Plate-forme selon l'une des revendications 1 ou 2, dans laquelle l'âme comprend deux couches (104, 106), dont l'une au moins a une structure en nid d'abeilles (108), lesdites couches étant reliées à une plaque intermédiaire.

5. Plate-forme selon l'une des revendications précédentes, dans laquelle au moins l'une des couches de revêtement (14, 16) est formée en un matériau composite stratifié.

6. Plate-forme selon l'une des revendications précédentes, dans laquelle au moins l'une des couches de revêtement est une feuille stratifiée comprenant un mat en fibres intérieur (24) et une mince plaque métallique extérieure (26).

7. Plate-forme selon l'une des revendications précédentes, comprenant un cadre périphérique (36, 38, 40, 42) inséré entre les couches de revêtement (14, 16), ledit cadre ayant une portion qui vient à l'affleurement des bords de la plate-forme (10).

8. Plate-forme selon la revendication 7, dans laquelle une fixation (30, 31) peut être rapportée sur ladite portion en affleurement dudit cadre.

9. Plate-forme selon la revendication 7 ou 8, dans laquelle une fixation (32, 80) peut être rapportée sur le cadre à travers l'une des couches de revêtement de la plate-forme.

10. Plate-forme selon la revendication 8 ou 9, dans laquelle une cabine d'ascenseur repose, en utilisation, sur ladite fixation (30).

11. Plate-forme selon l'une des revendications précédentes, dans laquelle des inserts (76, 82, 88, 96) servant à fixer des pièces à ladite plate-forme (10) sont noyés dans ladite âme (12) et s'étendent à travers au moins l'une desdites couches de revêtement (14, 16).

12. Plate-forme selon l'une des revendications précédentes, dans laquelle la plate-forme (10) convient pour être utilisée comme plate-forme d'ascenseur.
